# EUROPÄISCHE PATENTSCHRIFT

(11) **EP 1 097 701 B1**
(45) Veröffentlichungstag und Bekanntmachung des Hinweises auf die Patenterteilung: **13.05.2009**
(21) Anmeldenummer: 00122666.1
(22) Anmeldetag: 18.10.2000
(51) Int. Cl.: A61K 8/04, A61K 8/39, A61K 8/73, A61K 8/81, A61K 8/86

(54) **Haarkosmetische Zubereitung auf der Grundlage eines Gemisches aus kationischen Polymeren, Celluloseethern, Polyethylenglycolen und Silicontensiden**
Hair cosmetics on the basis of a mixture of catione polymers, cellulose ethers, polyethyleneglycols and silicone surfactants
Compositions cosmétiques capillaires à base d'un mélange de polymères cationiques et d'éthers de cellulose et de polyéthylène glycols et de tensioactifs siliconiques

(30) Priorität: 03.11.1999 DE 19952794
(43) Veröffentlichungstag der Anmeldung: 09.05.2001
(73) Patentinhaber: Beiersdorf AG, 20253 Hamburg (DE)
(72) Erfinder: Koller, Andreas, Dr., 21035 Hamburg (DE); Sass, Viola, 22880 Wedel (DE)

(56) Entgegenhaltungen:
- WO-A1-88/06878
- WO-A1-99/13837
- WO-A1-99/13846
- DE-A1- 4 234 743
- DE-A1- 19 531 145
- DE-A1- 19 534 722
- US-A- 5 514 369

## Beschreibung

Die vorliegende Erfindung betrifft haarkosmetische Wirkstoffe und Zubereitung zur Festigung, Formgebung, Kräftigung und Strukturverbesserung der Haare

Eine ansprechend aussehende Frisur wird heute allgemein als unverzichtbarer Teil eines gepflegten Äußeren angesehen. Dabei gelten aufgrund aktueller Modeströmungen immer wieder Frisuren als chic, die sich bei vielen Haartypen nur unter Verwendung bestimmter festigender Wirkstoffe aufbauen bzw. für einen längeren Zeitraum aufrechterhalten lassen.

Aus diesen Gründen werden seit geraumer Zeit Haarstylingprodukte verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Die Eigenschaft der Fülle wird einer Frisur beispielsweise zugeschrieben, wenn das Haar nach der Behandlung nicht flach auf der Kopfhaut aufliegt und gut frisierbar ist.

Die Eigenschaft des Volumen wird einer Frisur beispielsweise zugeschrieben, wenn das Haar nach der Behandlung Fülle und Sprungkraft aufweist.

Die Eigenschaft des Body's wird einer Frisur beispielsweise zugeschrieben, wenn das Haarvolumen selbst unter äußeren, störenden Einflüssen groß bleibt.

Es sind zahlreiche Haarverformungsmittel bekannt, die eine permanente Haarverformung bewirken, beispielsweise Dauerwellmittel. Dabei werden die Disulfidbrücken des Haarkeratins reduktiv gespalten und nach einer mechanischen Verformung durch Oxidationsmittel wieder verknüpft.

Im Gegensatze zu solchen "echten" Haarverformungsmitteln verändern Haarstylingprodukte das Haar chemisch nicht. Die Formulierungen wäßrig-alkoholischer Haarstylingmittel enthalten im allgemeinen bis zu 40 % Ethanol, n-Propanol oder Isopropanol. Als haarfestigende Substanz wird üblicherweise ein Filmbildner eingesetzt. Die Wahl des Filmbildners hängt von der beabsichtigten kosmetischen Eleganz der Formulierungen ab, im einfachsten Falle können sogar Zuckerlösungen (z.B. Maltose) verwendet werden. Heutzutage üblicher sind allerdings polymere Filmbildner, welche oftmals teil- oder vollsynthetischer Natur sind.

Festigende Wirkstoffe, bei denen es sich in der Regel um polymere Verbindungen handelt, können auch in übliche Haarreinigungs- oder -konditioniermittel eingearbeitet werden. In vielen Fällen ist es aber vorteilhaft, sie in Form spezieller Mittel wie Haarfestiger oder Haarsprays anzuwenden.

Die Zubereitungsformen, in welchen sich Haarstylingmittel verwirklichen lassen, tragen, je nach Anwendungsgebiet und Zusammensetzung, vielfältige Bezeichnungen: Haargele, Wet-Gele, Haarwachse, Setting Lotionen, Haarfestiger, Haar Styling Lotionen, Fönfestiger, Fänlotionen, Schaumfestiger, Haarsprays etc..

Es gibt nun in jüngster Zeit eine Reihe von Entwicklungen auf dem Haarkosmetikgebiet, die einen Bedarf an neuartigen festigenden Wirkstoffen bzw. neuen Formulierungsformen geweckt haben. Viele dieser Entwicklungen beruhen dabei nicht auf anwendungstechnischen Nachteilen oder Unzulänglichkeiten der bekannten Mittel, sondern z.B. auf Umweltschutzgesichtspunkten, gesetzlichen Auflagen oder anderen "nicht-technischen" Ursachen, wie z.B. der weitläufig bekannten negativen Verbrauchereinstellung bezüglich alkoholhaltiger haarkosmetischer Zubereitungen.

So wird insbesondere verstärkt ein Übergang von Mitteln auf Basis flüchtiger organischer Verbindungen (sogenannter "volatile organic compounds" oder auch kurz: VOC's), z.B. Alkoholen, zu Mitteln auf rein wäßriger Basis angestrebt.

Der Stand der Technik läßt es aber an Zubereitungen mangeln, welche den vorab genannten Anforderungen entsprechen. Die Haartracht fixierende Zubereitungen des Standes der Technik enthalten beispielsweise in der Regel Bestandteile (synthetische oder natürliche Polymere), welche Gefahr laufen, bei teilweisem oder vollständigem Ersatz leichtflüchtiger organischer Bestandteile durch Wasser eine signifikante Beeinträchtigung der Produkteigenschaften zu erfahren, was oft durch geschickte Formulierung kompensiert werden muß.

Es bestand also die Aufgabe, entsprechende alkoholarme, insbesondere alkoholfreie Mittel zu entwickeln, die hinsichtlich der anwendungstechnischen Eigenschaften, beispielsweise dem maximal erreichbaren Festigungsgrad und dem Langzeithalt, die vom Verbraucher gesteckten Erwartungen erfüllen und gleichzeitig einen reduzierten Anteil an flüchtigen organischen Verbindungen aufweisen, ohne daß die elementaren Eigenschaften des Polymerfilms auf den Haaren, wie z.B. Klarheit/Tranparenz, Oberflächentaktilität, Glanz und Auswaschbarkeit beeinträchtigt werden.

Erstaunlicherweise werden alle diese Aufgaben gelöst durch haarkosmetische Zubereitungen, enthaltend Wirkstoffkombinationen aus
(a) Poyquaternium-4,
(b) einem oder mehreren Celluloseethern
(c) einem oder mehreren Silikonemulgatoren, gewählt aus der Gruppe der grenzflächenaktive Substanzen aus der Gruppe der Verbindungen, welche gekennzeichnet sind durch die folgende chemische Struktur: 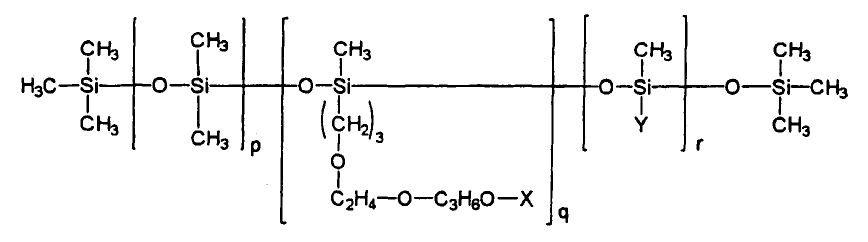 bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.
(d) einem oder mehreren Polyethylenglycolen

Die erfindungsgemäßen Zubereitungen verleihen der Haartracht lockere Fülle und Festigkeit bis hin zu sehr hohen Härtungsgraden und Langzeithalt, ohne klebrig zu wirken. Sie dienen der Erhöhung der Haarfülle, zur Verbesserung des Haarbodys und des Haarvolumens sowie des Haltes der Haartracht.
Polyquaternium-4 ist ein Copolymeres der Hydroxyethylcellulose mit Dimethyldiallylamoniumchlorid.

Polyquaternium-4, wird unter der Handelsbezeichnung Celquat^{®} H100 bzw. Celquat^{®} L200 von der Fa. National Starch angeboten.

Erfindungsgemäß enthalten haarkosmetische Zubereitungen 0,01 bis 50 Gew.%, vorteilhaft 0,1 bis 10 Gew.%, ganz besonders bevorzugt 0,25 bis 2,5 Gew.-% Polyquaternium 4.

Der oder die Celluloseether werden vorteilhaft gewählt aus der Gruppe der Substanzen, die sich durch die allgemeine Struktur 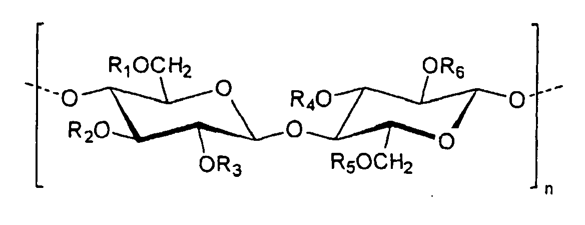 auszeichnen, wobei R₁ - R₆ unabhängig voneinander gewählt werden aus der Gruppe H, verzweigtes oder unverzweigtes C₁ - C₈ -Alkyl, wobei wenigstens einer der Reste R₁ -R₆ einen verzweigten oder unverzweigten C₁ - C₈ - Alkylrest darstellt, und n eine Zahl zwischen 200 und 1.000.000 darstellt.

Vorteilhafte Celluloseether sind beispielsweise Hydroxymethylcellulose, Hydroxyethylcellulose, Hydroxypropylcellulose, Hydroxypropylmethylcellulose, Hydroxybutylcellulose.

Erfindungsgemäß enthalten haarkosmetische Zubereitungen 0,01 bis 50 Gew.-%, vorteilhaft 0,1 bis 10 Gew.-%, ganz besonders bevorzugt 0,25 bis 2,5 Gew.-% eines oder mehrerer Celluloseether.

Ein Beispiel für besonders vorteilhaft im Sinne der vorliegenden Erfindung zu verwendende Silikonemulgatoren sind Dimethiconcopolyole, welche von der Gesellschaft Th.Goldschmidt AG unter den Warenbezeichnungen ABIL^{®} B 8842, ABIL^{®} B 8843, ABIL^{®} B 8847, ABIL^{®} B 8851, ABIL^{®} B 8852, ABIL^{®} B 8863, ABIL^{®} B 8873 und ABIL^{®} B 88183 bzw. unter der Warenbezeichnung Dow Corning^{®} Q2-5220, Dow Corning^{®} 190 und Dow Corning^{®} 193von der Gesellschaft Dow Corning Ltd. verkauft werden.

Ein weiteres Beispiel für besonders vorteilhaft im Sinne der vorliegenden Erfindung zu verwendende Silikonemulgatoren ist das Cetyl Dimethiconcopolyol, welches von der Gesellschaft Th.Goldschmidt AG unter der Warenbezeichnung ABIL^{®} EM 90 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an erfindungsgemäß verwendeten Siliconemulgatoren in den erfindungsgemäßen kosmetischen oder dermatologischen Zubereitungen wird vorteilhaft aus dem Bereich von 0,1 - 10,0 Gew.-%, bevorzugt 0,5 - 5,0 Gew.-% gewählt, bezogen auf das Gesamtgewicht der Zubereitungen.

Ganz besonders bevorzugt zeichnen sich die Zubereitungen gemäß der Erfindung durch einen zusätzlichen Gehalt an einem oder mehreren Polyethylenglycolen aus, insbesondere solcher der allgemeinen Formel 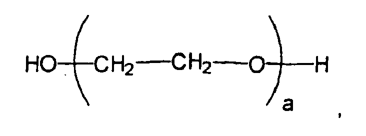 in welcher a vorteilhaft Werte zwischen 3 und 25 annimmt.

Die Gesamtmenge an erfindungsgemäß vorteilhaft verwendeten Polyethylenglycolen in den erfindungsgemäßen kosmetischen oder dermatologischen Zubereitungen wird vorteilhaft aus dem Bereich von 0,1 - 20,0 Gew.-%, bevorzugt 2 - 12,0 Gew.-% gewählt, bezogen auf das Gesamtgewicht der Zubereitungen.

Zur Anwendung werden die erfindungsgemäßen kosmetischen und dermatologischen Zubereitungen in der für Kosmetika üblichen Weise auf die Haare in ausreichender Menge aufgebracht.

In kosmetischen Zubereitungen zur Festigung der Haare, wie z.B. Haarsprays, Schaumfestiger, Flüssigfestiger, Stylinggele usw., können die erfindungsgemäß einzusetzenden Wirkstoffkombinationen vorzugsweise in Konzentrationen von 0,5 bis 30 Gewichtsprozent eingesetzt werden.

Es sind natürlich auch Mischungen mit allen grundsätzlich für diesen Zweck vorgeschlagenen und bekannten Polymere möglich, wobei die Einsatzkonzentrationen je nach Zubereitung, Polymertyp und Mischungsverhältnis in der Regel bevorzugt zwischen 0,5 und 10 Gewichtsprozent liegen.

Zur Mischung geeignet sind neben wasserlöslichen oder wasserdispergierbaren Polyurethanen und Silikonharzen beispielsweise die im folgenden aufgeführten nichtionische Polymere, sofern sie miteinander kompatibel sind. Die Mischungen der erfindungsgemäßen sulfonhaltigen Polyester mit einem oder mehreren der im folgenden aufgeführten Polymere werden bevorzugt in, wäßrigen Lösungen eingesetzt.

Es ist bevorzugt, den Gehalt an niederen Alkoholen (C< 5) geringer als 5 Gew.-%, bevorzugt geringer als 2 Gew.-%, besonders bevorzugt geringer als 1 Gew.-% zu wählen, wobei es insbesondere bevorzugt ist, im wesentlichen alkoholfreie Zubereitungen auszugestalten.

Nichtionische Polymere, die in den erfindungsgemäßen Zubereitungen zusammen mit den erfindungsgemäßen sulfonhaltigen Polyestern verwendet werden, sind insbesondere Vinylpyrrolidon-Homo- oder Copolymerisate. Dazu gehören Polyvinylpyrrolidon, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze.

Weitere erfindungsgemäß geeignete Copolymere sind solche aus Vinylpyrrolidon und Dimethylaminoethylmethacrylat,Terpolymere aus Vinylcaprolactam, Vinylpyrrolidon und Dimethylaminoethylmethacrylat, Polysiloxane usw.

Die erfindungsgemäßen Zusammensetzungen zur Festigung der Haare können als Haarsprays oder Schaumaerosole vorliegen und die dafür üblichen und dem Stand der Technik entsprechenden Zusätze enthalten, sofern eine entsprechende Kompatibilität vorliegt. Dies sind beispielsweise weitere Lösungsmittel wie niedere Polyalkohole und deren toxikologisch verträglichen Ether und Ester, Weichmacher, leicht- und schwerflüchtige Silikone, leicht- und schwerflüchtige verzweigte bzw. unverzweigte Kohlenwasserstoffe, Emulgatoren, Antioxidantien, Wachse Stabilisatoren, pH-Wert-Regulatoren, Farbstoffe, Konsistenzgeber, Antistatika, UV-Absorber, Parfums, usw.

Soll die erfindungsgemäße Zusammensetzung als Haarspray oder Schaumaerosol verwendet werden, so wird in der Regel ein Treibmittel zugesetzt. Übliche Treibmittel sind niedere Alkane, beispielsweise Propan, Butan oder Isobutan, Dimethylether, Stickstoff, Stickstoffdioxid oder Kohlendioxid oder Gemische aus diesen Substanzen.

Bei Verwendung in mechanischen Sprüh- oder Schaumvorrichtungen, beispielsweise Sprühpumpen oder manuellen Schaumpumpen bzw. Squeeze-systemen, kann das Treibmittel in der Regel entfallen.

Die wäßrigen erfindungsgemäßen Zubereitungen enthalten gegebenenfalls vorteilhaft Diole oder Polyole niedriger C-Zahl, sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrückenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = Gefrorenes" über den alchimistischen Ausdruck "gelatine" (16. Jhdt.) für nhdt. Gelatine"] durchaus gerecht werden.

Bei kosmetischen und dermatologischen Zubereitungen gemäß der Erfindung beispielsweise kann es sich beispielsweise auch um Zubereitungen zum Fönen oder Einlegen der Haare, Zubereitungen zum Färben, um eine Frisier- oder Behandlungslotion handeln.

Erfindungsgemäße Zubereitungen können sich gegebenenfalls vorteilhaft durch einen Gehalt an kationischen und/oder nichtionischen Tensiden auzeichnen.

Gegebenenfalls vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Eine kosmetische Zubereitung in Form einer Lotion, die nicht ausgespült wird, insbesondere eine Lotion zum Einlegen der Haare, eine Lotion, die beim Fänen der Haare verwendet wird, eine Frisier- und Behandlungslotion, stellt im allgemeinen eine wäßrige Lösung dar und enthält die erfindungsgemäßen Wirkstoffkombinationen.

Die erfindungsgemäßen Zusammensetzungen enthalten gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Rezepturbeispiele:

### A. Styling Fluids

| | Extra starker Halt | sehr starker Halt | starker Halt |
|---|---|---|---|
| | **1** | **2** | **3** |
| | Gew.% | Gew.% | Gew.% |
| Polyquaternium-4 | 2,00 | 1,50 | 1,00 |
| Hydroxyethylcellulose | 0,80 | 0,80 | 0,80 |
| Dimethiconcopolyol | 0,10 | 0,10 | 0,10 |
| Polyethylenglykol | 5,00 | 5,00 | 5,00 |
| Parfüm, Lösungsvermittler, pH-Einstellung, Konservierungsmittel | q.s. | q.s. | q.s. |
| Wasser, VES | ad 100,00 | | |

### B. Styling Gele

| | Extra starker Halt | sehr starker Halt | starker Halt |
|---|---|---|---|
| | **4** | **5** | **6** |
| | Gew.% | Gew.% | Gew.% |
| Polyquaternium-4 | 2,0 | 1,50 | 1,00 |
| Hydroxyethylcellulose | 1,00 | 1,00 | 0,20 |
| Dimethiconcopolyol , | 0,10 | 0,10 | 0,10 |
| Polyethylenglykol | 5,00 | 5,00 | 5,00 |
| Hydroxypropylguar | - | - | 0,80 |
| Parfüm, Lösungsvermittler, pH-Einstellung, Konservierungsmittel | q.s. | q.s. | q.s. |
| Wasser, VES | ad 100,00 | | |

### C. Schaumfestiger

| | Extra starker Halt | sehr starker Halt | Starker Halt |
|---|---|---|---|
| | **7** | **8** | **9** |
| | Gew.% | Gew.% | Gew.% |
| Polyquaternium-4 | 1,50 | 1,50 | 1,00 |
| Hydroxyethylcellulose | 0,05 | 0,05 | 0,05 |
| Dimethiconcopolyol | 0,10 | 0,10 | 0,10 |
| Polyethylenglykol | 0,50 | 0,50 | 0,50 |
| Cetyltrimethylammoniumchlorid | 0,20 | 0,20 | 0,20 |
| Parfüm, Lösungsvermittler, pH-Einstellung, Konservierungsmittel | q.s. | q.s. | q.s. |
| Propan/Butan | 10,00 | | |
| Wasser, VES | ad 100,00 | | |

### D. Schaumfestiger mit Wachseffekt

| | Extra starker Halt | Starker Halt | Normaler Halt |
|---|---|---|---|
| | **10** | **11** | **12** |
| | Gew.% | Gew.% | Gew.% |
| Polyquaternium-4 | 2.50 | 2.50 | 1.50 |
| Hydroxyethylcellulose | 0.35 | 0.35 | 0.35 |
| Dimethiconcopolyol | 0.15 | 0.15 | 0.15 |
| Polyethylenglykol | 15.00 | 15.00 | 15.00 |
| Cetyltrimethylammoniumchlorid | 0.05 | 0.05 | 0.05 |
| Parfüm, Lösungsvermittler, pH-Einstellung, Konservierungsmittel | q.s. | q.s. | q.s. |
| Propan/Butan | 10,00 | | |
| Wasser, VES | ad 100,00 | | |

## Patentansprüche

1. Haarkosmetische Zubereitungen, enthaltend Wirkstoffkombinationen aus
(a) Polyquater-Polyquaternium-4,
(b) einem oder mehreren Celluloseethern
(c) einem oder mehreren Silikonemulgatoren, gewählt aus der Gruppe der grenzflächenaktive Substanzen aus der Gruppe der Verbindungen, welche **gekennzeichnet sind durch** die folgende chemische Struktur: 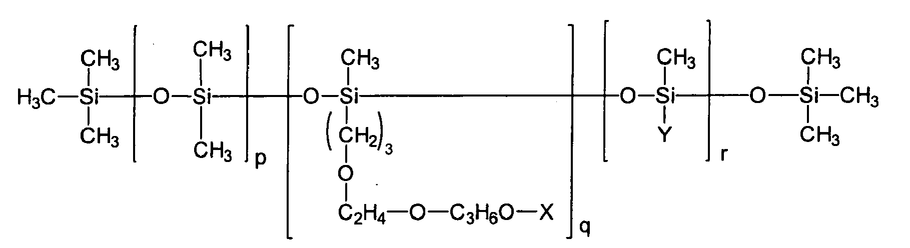 bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.
(d) einem oder mehren Polyethylenglycolen

2. Haarkosmetische Zubereitungen nach Anspruch 1, **dadurch gekennzeichnet, daß** sie 0,01 bis 50 Gew.-%, vorteilhaft 0,1 bis 10 Gew.-%, ganz besonders bevorzugt 0,25 bis 2,5 Gew.-% Polyquaternium 4 enthalten.

3. Haarkosmetische Zubereitungen nach Anspruch 1, **dadurch gekennzeichnet, daß** der oder die Celluloseether gewählt werden aus der Gruppe Hydroxymethylcellulose, Hydroxyethylcellulose, Hydroxypropylcellulose, Hydroxypropylmethylcellulose, Hydroxybutylcellulose.

4. Haarkosmetische Zubereitungen nach Anspruch 1, **dadurch gekennzeichnet, daß** sie 0,01 bis 50 Gew.-%, vorteilhaft 0,1 bis 10 Gew.-%, ganz besonders bevorzugt 0,25 bis 2,5 Gew.-% eines oder mehrerer Celluloseether enthalten.

5. Haarkosmetische Zubereitungen nach Anspruch 1, **dadurch gekennzeichnet, daß** der oder die Silikonemulgatoren gewählt wird aus der Gruppe Dimethiconcopolyol, Cetyl Dimethiconcopolyol, Cyclomethicon Dimethiconcopolyol, Laurylmethiconcopolyol.

6. Haarkosmetische Zubereitungen nach Anspruch 1, **dadurch gekennzeichnet, daß** sie 0,1 - 10,0 Gew.-%, bevorzugt 0,5 - 5,0 Gew.-% gewählt, bezogen auf das Gesamtgewicht der Zubereitungen eines oder mehrerer Silikonemulgatoren enthalten.

7. Haarkosmetische Zubereitungen nach Anspruch 1, durch einen zusätzlichen Gehalt an einem oder mehreren Polyethylenglycolen **gekennzeichnet**, insbesondere solcher der allgemeinen Formel 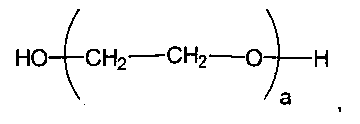 in welcher a vorteilhaft Werte zwischen 3 und 25 annimmt.

8. Haarkosmetische Zubereitungen nach Anspruch 7, **dadurch gekennzeichnet, daß** die Gesamtmenge an Polyethylenglycolen aus dem Bereich von 0,1 - 15,0 Gew.-%, bevorzugt 2,0 - 12,0 Gew.-% gewählt wird, bezogen auf das Gesamtgewicht der Zubereitungen.

## Claims

1. Hair cosmetic preparations comprising active ingredient combinations of
(a) polyquaternium-4,
(b) one or more cellulose ethers
(c) one or more silicone emulsifiers selected from the group of interface-active substances from the group of the compounds which are **characterized by** the following chemical structure: 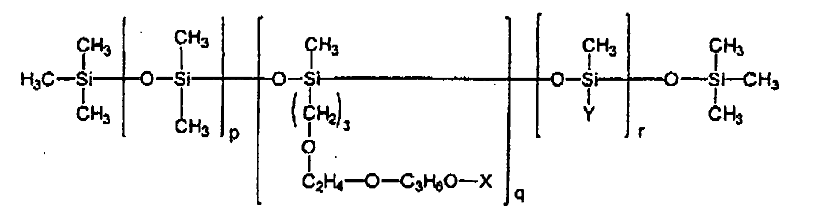 in which X and Y, independently of one another, are selected from the group H and the branched and unbranched alkyl groups, acyl groups and alkoxy groups with 1-24 carbon atoms, p is a number from 0-200, q is a number from 1-40, and r is a number from 1-100,
(d) one or more polyethylene glycols.

2. Hair cosmetic preparations according to Claim 1, **characterized in that** they comprise 0.01 to 50% by weight, advantageously 0.1 to 10% by weight, very particularly preferably 0.25 to 2.5% by weight of polyquaternium-4.

3. Hair cosmetic preparations according to Claim 1, **characterized in that** the cellulose ether or the cellulose ethers are selected from the group consisting of hydroxymethylcellulose, hydroxyethylcellulose, hydroxypropylcellulose, hydroxypropylmethylcellulose, hydroxybutylcellulose.

4. Hair cosmetic preparations according to Claim 1, **characterized in that** they comprise 0.01 to 50% by weight, advantageously 0.1 to 10% by weight, very particularly preferably 0.25 to 2.5% by weight, of one or more cellulose ethers.

5. Hair cosmetic preparations according to Claim 1, **characterized in that** the silicone emulsifier or emulsifiers are selected from the group consisting of dimethicone copolyol, cetyl dimethicone copolyol, cyclomethicone dimethicone copolyol, lauryl methicone copolyol.

6. Hair cosmetic preparations according to Claim 1, **characterized in that** they comprise 0.1-10.0% by weight, preferably 0.5-5.0% by weight, based on the total weight of the preparations, of one or more silicone emulsifiers.

7. Hair cosmetic preparations according to Claim 1, **characterized by** an additional content of one or more polyethylene glycols, in particular those of the general formula 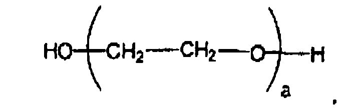 in which a advantageously assumes values between 3 and 2.5.

8. Hair cosmetic preparations according to Claim 7, **characterized in that** the total amount of polyethylene glycols is selected from the range from 0.1-15.0% by weight, preferably 2.0-12.0% by weight, based on the total weight of the preparations.

## Revendications

1. Compositions cosmétiques pour cheveux, contenant des combinaisons de substances actives constituées par
(a) du polyquaternium-4
(b) un ou plusieurs éthers de cellulose
(c) un ou plusieurs émulsifiants de silicone, choisis dans le groupe des substances tensioactives du groupe formé par les composés qui sont **caractérisés par** la structure chimique suivants : 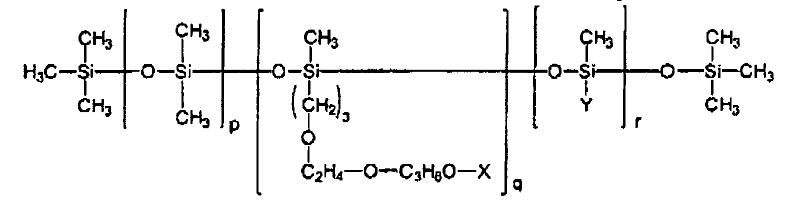 dans laquelle X et Y sont choisis, indépendamment l'un de l'autre, dans le groupe formé par H ainsi que les groupes alkyle, acyle et alcoxy ramifiés et non ramifiés, comprenant 1-24 atomes de carbone, p représente un nombre de 0-200, q représente un nombre de 1-40, et r représente un nombre de 1-100.
(d) un ou plusieurs polyéthylèneglycols.

2. Compositions cosmétiques pour cheveux selon la revendication 1, **caractérisées en ce qu'**elles contiennent 0,01 à 50% en poids, avantageusement 0,1 à 10% en poids, de manière tout particulièrement préférée 0,25 à 2,5% en poids de polyquaternium-4.

3. Compositions cosmétiques pour cheveux selon la revendication 1, **caractérisées en ce que** le ou les éthers de cellulose sont choisis dans le groupe formé par l'hydroxyméthylcellulose, l'hydroxyéthylcellulose, l'hydroxypropylcellulose, l'hydroxypropylméthylcellulose, l'hydroxybutylcellulose.

4. Compositions cosmétiques pour cheveux selon la revendication 1, **caractérisées en ce qu'**elles contiennent 0,01 à 50% en poids, avantageusement 0,1 à 10% en poids, de manière tout particulièrement préférée 0,25 à 2,5% en poids d'un ou de plusieurs éthers de cellulose.

5. Compositions cosmétiques pour cheveux selon la revendication 1, **caractérisées en ce que** le ou les émulsifiants de silicone sont choisis dans le groupe formé par le diméthiconecopolyol, le cétyldiméthiconecopolyol, le cyclométhicone-diméthiconecopolyol, le laurylméthiconecopolyol.

6. Compositions cosmétiques pour cheveux selon la revendication 1, **caractérisées en ce qu'**elles contiennent 0,1-10,0% en poids, de préférence 0,5-5,0% en poids, par rapport au poids total des compositions d'un ou de plusieurs émulsifiants de silicone.

7. Compositions cosmétiques pour cheveux selon la revendication 1, **caractérisées par** une teneur supplémentaire en un ou plusieurs polyéthylèneglycols, en particulier ceux dé formule générale 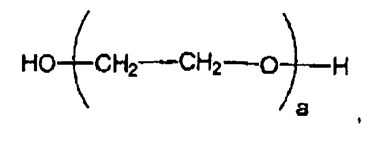 dans laquelle a présente avantageusement des valeurs entre 3 et 25.

8. Compositions cosmétiques pour cheveux selon la revendication 7, **caractérisées en ce que** la quantité totale de polyéthylèneglycols est choisie dans la plage de 0,1-15,0% en poids, de préférence de 2,0-12,0% en poids, par rapport au poids total des compositions.
